(19)

Europäisches
Patentamt

European
Patent Office

Office européen
des brevets

(11) **EP 4 414 707 A1**

(12) **EUROPEAN PATENT APPLICATION**

(43) Date of publication:
**14.08.2024 Bulletin 2024/33**

(21) Application number: **23156155.6**

(22) Date of filing: **10.02.2023**

(51) International Patent Classification (IPC):
**G01N 33/553** (2006.01)     **G01N 33/569** (2006.01)

(52) Cooperative Patent Classification (CPC):
**G01N 33/56911; G01N 33/553;** G01N 2333/01;
G01N 2800/44

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Technische Universität München
80333 München (DE)**

(72) Inventors:
• **VOGELE, Kilian
82399 Raisting (DE)**
• **SIMMEL, Friedrich
80336 München (DE)**

(74) Representative: **Schiweck Weinzierl Koch
Patentanwälte Partnerschaft mbB
Ganghoferstraße 68 B
80339 München (DE)**

(54) **METHOD FOR DETERMINING BINDING OF A TAIL FIBER PROTEIN TO THE SURFACE OF A CELL**

(57)   The invention relates to a method for determining binding of a tail fiber protein to a cell surface epitope of a bacterium using tail fiber protein-coated nanoparticles that undergo plasmonic coupling upon binding to the bacterial epitope. The invention further relates to the use of said nanoparticles for the method described above. Moreover, the invention relates to a method for determining promising bacteriophage candidates for antimicrobial treatment using said nanoparticles. The invention further relates to a kit for detecting plasmonic coupling.

EP 4 414 707 A1

**Description**

**FIELD OF THE INVENTION**

The invention relates to a method for determining binding of a tail fiber protein to a cell surface epitope of a bacterium using tail fiber protein-coated nanoparticles that undergo plasmonic coupling upon binding to the bacterial epitope. The invention further relates to the use of said nanoparticles for the method described above. Moreover, the invention relates to a method for determining promising bacteriophage candidates for antimicrobial treatment using said nanoparticles. The invention further relates to a kit for detecting plasmonic coupling.

**BACKGROUND OF THE INVENTION**

Bacteriophages are viruses that specifically infect a host bacterium (hereinafter "host bacterium") and replicate at the expense of that bacterium. The biotechnological applications of bacteriophages are very broad and include the use of bacteriophages themselves as a substitute for antibiotics in so-called bacteriophage therapy (Barbu et al. 2016). The latter is based on the natural property of bacteriophages to specifically attack and destroy pathogenic bacteria (lysis). Due to this specificity, phages are usually used as therapeutic agents in a personalized manner to generate higher chances of success. This approach to combat microbial infections, which has already been pursued for quite a long time, has been receiving more and more attention in recent years, as the number and spread of multidrug-resistant germs has greatly increased worldwide and the number of new antibiotics cannot keep up with this development. Bacteriophages also play an important role in the food industry, where they are used, for example, to detect and prevent listeria infestation of food (Strydom and Witthuhn 2015).

The surface structure of bacteria is an important factor in determining whether a bacterium is pathogenic or not. Enterohemorrhagic Escherichia coli (EHEC), which often has one of the following three serogroups 0157, 0103 and 026 is highly pathogenic where in contrast other E. coli are an important part of the microbiome. These serogroups represent the respective surface antigen acting lipopolysaccharides of the outer cell membrane of the bacteria. The surface structures recognized there are the Shiga-like toxins that are decisive for pathogenicity.

Besides the determination of the particular bacterium, the determination of the surface structure is of interest for the binding of phages. This determination plays a decisive role in personalized phage therapy.

The resonant frequency of metallic nanoparticles is in the visible range of light and is sensitive to the dielectric constant of the environment. The absorption wavelength of metal nanoparticles depends on their size, shape, and orientation and distance with respect to each other (Goncalves 2014). The effect of this resonance can be explained with the help of particle plasmons, which are collective oscillations of electrons in the conduction band of the material (Tan et al. 2011). The model of particle plasmons is based on the fact that electromagnetic waves deflect the electrons relative to the stationary atomic hulls in the particles. For this to happen, the diameter of the particles must match that of the penetration depth of that wave. The positive charge forms a restoring force for the electrons and thus this system can be seen as a driven harmonic oscillator. The particle plasmons are thus oscillations of those electrons and are called quasi-particles.

**OBJECTIVES AND SUMMARY OF THE INVENTION**

Therefore there is a need for a swift and less laborious general applicable method for the highly specific determination of bacterium species and discrimination of specific bacteria serogroups.

To solve this problem, the inventors established a method in which nanoparticles coated with tail fibers specific for a certain bacterium undergo easily detectable plasmonic coupling when the tail fibers bind to their respective epitopes by clustering on the surface of the bacterium.

Thus, a first aspect of the invention refers to a method for determining binding of a tail fiber protein to a cell surface epitope of a bacterium, comprising the steps of:

  a) contacting a plasmonic nanoparticle coated with a tail fiber protein with the bacterium in an aqueous solution to obtain a mixed solution;
  b) determining the absorption spectrum of the mixed solution;

wherein a shift in the absorption spectrum indicates binding of the tail fiber protein to the cell surface epitope of the bacterium thereby reducing the distance between the coated plasmonic nanoparticles to allow for plasmon coupling.

Hence, the invention exploits plasmonic coupling between nanoparticles coated by tail fibers, which can occur through clustering of the nanoparticles when specifically binding to the surface of a bacterium via the tail fiber protein.

Tail fibers allow for a highly specific detection of bacterial epitopes. Due to lack of glycosylation they are easy to produce and to modify in bacteria or in cell-free expression systems.

The readout of the shift in the absorption spectrum upon binding to the bacterium allows for a simple and fast detection. Hence, the assay of the invention provides a straightforward, quick and highly specific assay for the detection of bacteria, which can distinguish between serogroups which differ in epitopes on the cell surface.

The shift in the absorption spectrum can be detected by visual inspection or spectrophotometry. Thus, there is no need for expensive and/or large instruments used for the methods of the invention.

A second aspect of the invention refers to the use of a plasmonic nanoparticle coated with a tail fiber protein for determining the binding of the tail fiber protein to a cell surface epitope of a bacterium by determining plasmon coupling between the plasmonic nanoparticles.

Thus, the invention does not rely on coating a metal plate with phages and measuring surface plasmon resonance after excitation by lasers.

A third aspect of the invention refers to a method for determining specific bacteriophage candidates for antibiotic treatment, comprising the steps of:

a) contacting a plasmonic nanoparticle coated with a viral tail fiber protein with a solution containing target bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for antibiotic treatment of the target bacteria.

Hence, the invention allows for rapid and highly specific identification of bacteriophage strains able to neutralize specific bacterium strains isolated from sources like patients, environmental samples, foods or beverages.

A fourth aspect of the invention refers to a kit for detection of plasmon coupling comprising:

- plasmonic nanoparticles coated with a tail fiber protein,
- an aqueous solution.

In other words, the invention refers to methods, and kits for rapid determination of binding of a tail fiber protein to the respective epitope.

**FIGURE LEGENDS**

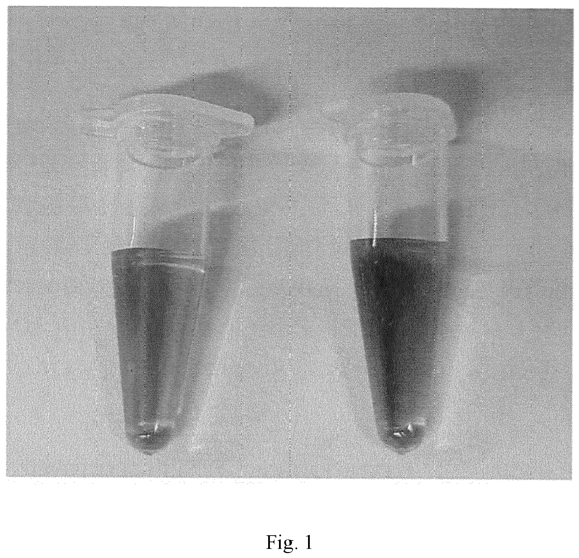

**Fig 1. Redshift occurring after contacting gold nanoparticles coated with tail fibers specific for ATCC 11303 with the respective bacteria.** Gold nanoparticles coated with a tailfiber as described in the Examples are present in both vials. The left vial shows the coated gold nanoparticles in a solution without the ATCC 11303 bacteria present. The solution has a red hue. The right vial shows the coated gold nanoparticles in a solution with the ATCC 11303 bacteria present, leading to a redshift in the absorption spectrum of the solution due to aggregation of the coated nanoparticles on the cell surface of the bacteria. The solution has a blue hue.

**DETAILED DESCRIPTION OF THE INVENTION**

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm 10\%$, and preferably of $\pm 5\%$.

Technical terms are used by their common sense. If a specific meaning is conveyed to certain terms, definitions of terms will be given in the following in the context of which the terms are used.

A first aspect of the invention refers to a method for determining binding of a molecule to a cell surface epitope, comprising the steps of:

a) contacting a plasmonic nanoparticle coated with a molecule capable of binding a cell surface epitope with a cell in an aqueous solution to obtain a mixed solution;
b) determining the absorption spectrum of the mixed solution;

wherein a shift in the absorption spectrum indicates binding of the molecule to the cell surface epitope thereby reducing the distance between the coated plasmonic nanoparticles to allow for plasmon coupling.

In one embodiment, the invention provides a method for determining binding of a tail fiber protein to a cell surface epitope of a bacterium, comprising the steps of:

a) contacting a plasmonic nanoparticle coated with a tail fiber protein with the bacterium in an aqueous solution to obtain a mixed solution;
b) determining the absorption spectrum of the mixed solution;

wherein a shift in the absorption spectrum indicates binding of the tail fiber protein to the cell surface epitope of the bacterium thereby reducing the distance between the coated plasmonic nanoparticles to allow for plasmon coupling.

In other words, the binding of the molecule to the cell surface epitope of the cell reduces the distance between the coated plasmonic nanoparticles. The reduced distance between the coated plasmonic nanoparticles leads to plasmon coupling which causes a shift in the absorption spectrum of the mixed solution.

Where the term "to cluster" is used in the present description, embodiments and claims, it refers to a group of similar matter positioned or occurring closely together, sometimes surrounding something. In some embodiments of the invention, plasmonic nanoparticles coated with a tail fiber protein cluster at their respective epitope on the surface of a cell.

In some embodiments of the present invention, step a) is performed before step b). In other embodiments, step b) may additionally be performed before step a). In other words, in some embodiments the absorption spectrum of the aqueous solution is determined before obtaining the mixed solution of step a), which is then followed by step b).

Where the term "nanoparticle" is used in the present description, embodiments and claims, it generally refers to a particle having a diameter in the range of about 1 nm to about 1000 nm. The nanoparticle may have various shapes. The shape of the nanoparticle may be shaped like a sphere, a triangle, a square, a cube, a pentagon, a hexagon, a heptagon, an octagon, a nonagon, a rhombus, an oval, a cross, a star, a diamond, a ring, a rod or any other form. Preferably, the nanoparticle is shaped like a sphere. Preferably, the nanoparticle is a metallic nanoparticle.

More preferably, the metal nanoparticle is a plasmonic nanoparticle. The plasmonic nanoparticle may be selected from the group consisting of a gold nanoparticle, a silver nanoparticle, a copper nanoparticle, a zinc nanoparticle, an aluminum nanoparticle, a bismuth nanoparticle, an MXene nanoparticle, a TiN nanoparticle, a tungsten nanoparticle and a platinum nanoparticle, optionally mixtures thereof and optionally nanoparticles comprising any one or more of the metals listed above. Preferably, the plasmonic nanoparticle is a gold nanoparticle. In one embodiment, the plasmonic nanoparticle is smaller than 1000 nm. In a preferred embodiment, the plasmonic particle is smaller than 200 nm.

In one embodiment, the plasmonic nanoparticle coated with a molecule capable of binding a cell surface epitope is the only type of plasmonic nanoparticle needed for determining binding of the molecule to the cell surface epitope. In other words, there is no need for a second type of plasmonic nanoparticle coated with a different molecule than the molecule the first type of plasmonic nanoparticle is coated with.

Where the term "molecule" is used herein, it refers in particular to a binding molecule capable of binding to the surface of a cell, in particular to an epitope of the cell. Examples for a molecule include, but are not limited to, a nucleic acid, a fatty acid, an amino acid or a peptide or protein, such as antibodies or binding fragments thereof, nanobodies or tail fibers. In one embodiment, the molecule capable of binding a cell surface epitope is a protein. Where the term "recombinant" is used in the present description, embodiments and claims, when used with a reference, e.g., to a cell, or nucleic acid, protein, or vector, it indicates that the cell, nucleic acid, protein or vector, has been modified by the introduction of a heterologous nucleic acid or protein or the alteration of a native nucleic acid or protein, or that the protein is derived from a nucleic acid so modified. In one embodiment, the recombinant protein is an antibody or a tail fiber protein.

Where the term "cell" is used in the present description, embodiments and claims, it refers to a single cell as well as to a population of cells. The cell may be any prokaryotic or eukaryotic cell. Such cells include, for example, a bacterium (such as *E .coli),* an archaea, a plant cell, an insect cell, a fungi cell, a yeast cell or a mammalian cell.

Where the term "cell surface" is used in the present description, embodiments and claims, it refers to the outermost layer of the cell that is accessible for binding without disturbing the cell's integrity. Typically, that outermost layer of the cell is a cell wall, cell membrane or plasma membrane but can also be a capsule in some types of cells.

Where the term "epitope" is used in the present description, embodiments and claims, it refers to any molecular structure on a cell surface to which a tail fiber protein can bind. Such molecular structures on a cell surface usually consist of chemically active surface groupings of molecules such as amino acids or sugar side chains and usually have specific three-dimensional structural characteristics, as well as specific charge characteristics. In some embodiments, an epitope may be an area of surface exposed residues and/or carbohydrate moieties on an antigen such as a lipopolysaccharide, polysaccharide or protein receptor on the cell surface.

Where the term "aqueous solution" is used in the present description, embodiments and claims, it refers to a solution in which the solvent is water. Preferably, the aqueous solution provides physiological conditions. In some embodiments, the aqueous solution has a pH value between 5 and 9, preferably a pH value between 6 and 8. In some embodiments, the aqueous solution has optical properties that allow for spectrophotometric measurements of the absorption spectrum of the solution. In some embodiments, the aqueous solution may be bodily fluids such as intracellular fluids or extracellular fluids, which comprise, but are not limited to, full blood, intravascular fluid (blood plasma), interstitial fluid, lymphatic fluid, transcellular fluid, cerebrospinal fluid, peritoneal fluid, pleural fluid, or amniotic fluid. In some embodiments, the bodily fluids may be diluted with water or a different physiological aqueous solution. In some embodiments, the aqueous solution can be a liquid potentially suitable for mammalian consumption such as water, river water, juices, or preparations of solid foods in liquefied form or mixed in water.

The term "contacting" as used herein means subjecting one matter to, and/or exposing to, a different matter. In some embodiments of the invention, plasmonic nanoparticles coated with a tail fiber protein are contacted with bacteria in an aqueous solution.

Where the term "tail fiber protein" is used in the present description, embodiments and claims, it refers to elongated proteins that self-assemble to form multimers and are capable of specific recognition of epitopes on cell surfaces which in nature are parts of bacteriophages that recognize bacterial epitopes. Compared to antibodies, for example, the tail fiber proteins possess three binding sites complementary to their respective epitope. The tail fiber protein may be a recombinant tail fiber protein. In some embodiments of the invention, a recombinant tail fiber protein is an engineered recombinant tail fiber protein capable of binding a cell surface epitope of a mammalian cell. In other embodiments of the invention, the tail fiber protein is a recombinant tail fiber protein derived from a naturally occurring tail fiber protein from a bacteriophage. Bacteriophage-derived tail fiber proteins typically bind to particular epitopes of lipopolysaccharides, polysaccharides or protein receptors on the cell surface. Sequences needed for the production of such tail fiber proteins are readily available in the Gene Ontology Resource database under accession number GO: 0098024.

Where the term "bacteriophage" is used herein, it refers to virus that infects bacteria or archaea. It is composed of capsid proteins that encapsulate a DNA or RNA genome. After infection of their genome into the cytoplasm, bacteriophages replicate in the microorganism using the transcription and translation apparatus of the bacterium. Phages are classified by the international Committee on Taxonomy of Viruses according to morphology and nucleic acid, including *Ackermannviridae, Myoviridae, Siphoviridae, Podoviridae, Lipothrixviridae, Rudiviridae, Ampullaviridae, Bicaudaviridae, Clavaviridae, Corticoviridae, Cystoviridae, Fuselloviridae, Globuloviridae, Inoviridae, Leviviridae, Microviridae, Plasmaviridae, Pleolipoviridae, Portogloboviridae, Spharolipoviridae, Spiraviridae, Tectiviridae, Tristromaviridae, Turriviridae.*

In some embodiments of the invention, the tail fiber protein comprises a purification tag. The nature of a tag will depend on the particular affinity purification system used. Various systems are available. In one embodiment, the affinity chromatographic system is immobilized metal affinity chromatography (IMAC), which is based on binding of a tag to a metal ion resin. Metal ions can be, e.g., zinc, nickel, or cobalt ions. The tag can be a polyhistidine sequence, which interacts specifically with metal ions such as nickel, cobalt, iron, or zinc. In some embodiments, the tag can be a polyhistidine sequence, which interacts specifically with metal ions such as zinc, nickel, or cobalt ions. A polyhistidine tag can be 2, 3, 4, 5, 6, 7, 8, 9, 10, 11, 12, 13, 14, 15, 16, 17, 18, 19, 20, 25, 30, 35, 40, or 50×His or other, provided that it binds essentially specifically to a metal ion. In a specific embodiment, the His tag is a 6xHis tag. The tag can also be a polylysine or polyarginine sequence, comprising at least four lysine or four arginine residues, respectively, which interact specifically with zinc, copper or a zinc finger protein.

Commercially available systems for IMAC include, but are not limited to, the following systems, which are sold as kits and as individual components, e.g., vectors, bacterial strains, affinity resins and instructions for use: HisTrap™ Fast Flow (Cytiva, formerly GE-Healthcare, US), QIAexpress Ni-NTA Protein Purification System of Qiagen (Qiagen, CA); HAT™ Protein Expression & Purification System (Clontech, Palo Alta, Calif.); pTrcHis Xpress™ Kit (InVitrogen); and BugBuster™ HisEind® Purification Kit (Novagen).

The term "bacterium" as used herein, refers to any one of the domain *Bacteria,* that comprises but is not limited to of round, spiral, or rod-shaped single-celled prokaryotic microorganisms that typically live in soil, water, organic matter, or the bodies of plants and animals, that make their own food especially from sunlight or are saprophytic or parasitic, are often motile by means of flagella, reproduce especially by binary fission, and include many important pathogens. In some embodiments, the bacterium is a multidrug resistant bacterium. Multidrug resistant bacteria are bacteria that have become resistant to treatment with antibiotics or other commonly used chemotherapeutic agents. Some bacterium strains have become resistant to practically all of the commonly available agents. Multidrug resistant bacteria comprise, but are not limited to, bacterium strains like Vancomycin-Resistant Enterococci, Methicillin-resistant Staphylococcus aureus, Extended-spectrum beta-lactamase producing Gram-negative bacteria, Klebsiella pneumoniae carbapenemase producing Gram-negative bacteria, Multidrug-resistant Gram-negative rods or Multidrug-resistant tuberculosis bacteria.

In some embodiments, the cell may be a mammalian cell. "Mammalian cell" refers to any cell derived from any animal of the class *Mammalia,* a large class of warm-blooded vertebrates having mammary glands in the female, a thoracic diaphragm, and a four-chambered heart. The class includes, but is not limited to, whales, carnivores, rodents, bats and primates. In further embodiments, the mammalian cell is a cancer cell or a tumor cell.

In a specific embodiment, the invention relates to a method for determining binding of a recombinant tail fiber protein engineered to bind to an epitope on the cell surface of a mammalian cell, comprising the steps of:

  a) contacting a plasmonic nanoparticle coated with the recombinant viral tail fiber protein engineered to bind to an epitope on the cell surface of a mammalian cell with said mammalian cell in an aqueous solution to obtain a mixed solution;
  b) determining the absorption spectrum of the mixed solution;

wherein a shift in the absorption spectrum indicates binding of the recombinant tail fiber protein engineered to bind to an epitope on the cell surface of a mammalian cell to said epitope, thereby reducing the distance between the coated plasmonic nanoparticles to allow for plasmon coupling.

Where the term "absorption spectrum" is used in the present description, embodiments and claims, it refers to the spectrum formed by electromagnetic radiation that has passed through a solution in which radiation of certain frequencies is absorbed. Various methods and systems for determining the absorption spectrum of a solution are known. These methods include, but are not limited to, visual inspection and spectrophotometry. Visual inspection refers to determination by eye. Spectrophotometry refers to determination by using a spectrophotometer which can measure the reflection or transmission properties of a material as a function of wavelength.

Where the term "determining" is used in the present description, embodiments and claims, it refers to ascertaining or establishing e.g. the absorption spectrum exactly by observation or measurement. In some embodiments, the absorption spectrum is determined by visual inspection or spectrophotometry. In some embodiments, a shift in the absorption spectrum is determined by determining the absorption spectrum before certain steps of the inventive methods and comparing them with the absorption spectrum after said steps of the inventive methods. In other embodiments, a shift in the absorption spectrum is determined by determining the absorption spectrum of a control and comparing it to the absorption spectrum of a sample treated according to the methods of the present invention.

Where the term "shift in the absorption spectrum" is used in the present description, embodiments and claims, it refers to a change in the determined or observed absorption spectrum of a solution. In some embodiments, the shift in the absorption spectrum may be a blueshift or a redshift. Preferably, the shift in the extinction spectra is a red shift.

Where the term "control" is used in the present description, embodiments and claims, it refers to an object or system that is not changed or modified, so that you can compare it with similar objects or systems that were intentionally changed or modified. In one embodiment, a control is an uncoated plasmonic nanoparticle. In another embodiment, a control is a plasmonic nanoparticle that is coated with a molecule that is not capable of binding the cell surface epitope that the coated plasmonic nanoparticle of the methods of the present invention can bind. In another embodiment, a control is an aqueous solution with the same optical properties as the aqueous solution of the methods of the present invention. In another embodiment, a control is a predetermined absorption spectrum value.

Where the term "plasmon coupling" is used in the present description, embodiments and claims, it refers to a phenomenon that occurs when two or more plasmonic particles approach each other to a distance below approximately one plasmonic particle diameter's length. Upon the occurrence of plasmon coupling, the resonance of individual particles start to hybridize, and their resonance spectrum peak wavelength will shift (either blueshift or redshift), depending on how surface charge density distributes over the coupled particles. At a single particle's resonance wavelength, the surface charge densities of close particles can either be out of phase or in phase, causing repulsion or attraction and thus leading to increase (blueshift) or decrease (redshift) of hybridized mode energy. The magnitude of the shift, which can be the measure of plasmon coupling, is dependent on the interparticle gap as well as particles geometry and plasmonic resonances supported by individual particles.

In the present example, the coupling of particle plasmons shifts the color of the solution from red to blue due to particle plasmon coupling. The oscillating electron gas can be described using a Lagrangian function. For this, only the temporal charge density $\sigma$ has to be calculated *via* the flux $\vec{j}$ of the electrons. This results from the deflection of the electrons S, which can be described with the help of a driven harmonic oscillator. Put together, the Lagrangian function then results in the following equation.

$$L = \frac{n_0 m_e}{2} \sum_{l,m} \left[ \dot{S}_{lm}^2 - \omega_l^2 S_{lm}^2 \right]$$

Herein, $n_0$ represents the electron count and $m_e$ the electron mass, with

$$\omega_l = \sqrt{\frac{4\pi e^2 n_0}{m_e}} \sqrt{\frac{l}{2l+1}}$$

as the mode of the oscillation. This can be extended for a dimer, which explains the shift of the absorption wavelength. For this, the driven harmonic oscillator is expanded by an interaction term in the potential part of the Lagrangian function. This results then to:

$$L = \frac{n_0 m_e}{2} \sum_{i,j} \left[ \left[ \dot{S}_i^2 - \omega_i^2 S_i^2 \right] \delta_{i,j} - \frac{\omega_B^2}{4\pi} V_{ij}^{(m)}(D) S_i S_j \right]$$

The interaction term results from solving the Laplace equation. The interaction term is zero if i and j belong to the same sphere. The interaction term is given by:

$$V_{ij}^{(m)} = 4\pi \sqrt{l_i l_j R_i^{2l_i+1} R_j} \int d\theta_j \cdot \sin\theta_j \cdot \frac{P_{l_i}^m(\cos\theta_j)}{(2l_i+1) X_i(\theta_j)^{l_i+1}} \cdot P_{l_i}^m(\cos\theta_j)$$

Where $P_{l_i}^m$ are the spherical surface functions and $X_i(\theta_j)^{l_i+1}$ is the distance from the surface of one plasmonic particle to the center of the other, as a function of the angle. There is a measurable coupling of the plasmons, if the distance of both is not greater than 2.5 times the distance of the radius of the particles (Tan et al. 2011).

In some embodiments, plasmon coupling occurs between plasmonic nanoparticles coated with a tail fiber protein that cluster at their respective epitope on the surface of a cell.

The present invention further relates to a method for determining specific bacteriophage candidates for antibiotic treatment, comprising the steps of:

a) contacting a plasmonic nanoparticle coated with a viral tail fiber protein with an aqueous solution containing target bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for antibiotic treatment of the target bacteria.

Where the term "antibiotic treatment" is used in the present description, embodiments and claims, it refers to treating, inhibiting or ameliorating a pathogenic bacterial infection or infestation. In one embodiment, the antibiotic treatment is a personalized antibiotic treatment. In other words, the treatment is not a general treatment, but is highly specific for the bacterium strain or bacterium strains that the patient is infected with.

In a preferred embodiment, the invention relates to a method for determining patient-specific bacteriophage candidates for personalized antibiotic treatment comprising the steps of:

a) contacting a plasmonic nanoparticle coated with a viral tail fiber protein with an aqueous solution containing patient-derived bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for personalized antibiotic treatment of the patient.

Where the term "patient" is used in the present description, embodiments and claims, it refers to an animal.

Animals comprise e.g. a human, a domestic animal, a livestock animal, or an exotic animal. In some embodiments, the patient is a human, a domestic animal, or a livestock animal. Preferably, the patient is a human.

The present invention also provides a kit comprising plasmonic nanoparticles coated with a tail fiber protein and an aqueous solution. Preferably, the kit further comprises one or more controls, which is/are selected from the group consisting of uncoated plasmonic nanoparticles and plasmonic nanoparticles coated with a tail fiber protein with specificity for a different cell surface epitope than the non-control plasmonic particle coated with a tail fiber protein. Preferably, the aqueous solution provided with the kit is suitable for absorption spectrum measurements. The kit may further comprise instructions for use.

In some embodiments, the plasmonic nanoparticle coated with a tail fiber protein is the only type of plasmonic nanoparticle needed for determining binding of the tail fiber protein to the cell surface epitope of a bacterium.

In some embodiments, the methods of the present invention do not involve surface-enhanced Raman spectroscopy. In some embodiments, the methods of the present invention do not involve measuring surface plasmon resonance.

The present invention further provides a plasmonic nanoparticle coated with a molecule capable of binding a cell surface epitope. In a specific embodiment, a plasmonic nanoparticle coated with a tail fiber protein capable of binding a cell surface epitope on a bacterium is provided.

While the invention has been illustrated and described in detail in the figures and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the figures, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

## EXPERIMENTS

### Example 1: Tail Fiber Protein Production

For the bacteriophage tail fiber protein T7p52, the gene was equipped with an N-terminal His-Tag (6xHis) and a cysteine residue and was then cloned into a pET20b(+) expression vector (Novagen) and transformed into the BL21(DE3)pLysS strain of *E. coli*. Expression was performed in a shake flask at 37 °C in a 1-liter culture in LB (Luria/Miller) medium (10 g tryptone, 5 g yeast extract, 10 g NaCl, and 100 mg carbenicillin) and induced with 240 mg IPTG (isopropyl-P-D-1-thiogalactopyranoside) once the optical density as measured at 600 nm reached 0.8. After 16 hours of incubation at 16 °C, the bacteria were harvested by centrifugation. Bacteria were lysed by sonication in phosphate-buffered saline (PBS, pH 7.4) supplemented with lysozyme (1 mg/mL), 1 mM pMSF, 1 mM benzamidine, and 0.5 U DNase 1. After lysis, 2 mL of 10% (w/v) PEI was added per 1 L of the original cell culture volume. Samples were further lysed on ice by ultrasonication for 10 min (50% amplitude, 30 sec pulse and 15 sec pause), followed by centrifugation at 16,000 $\times$ g at 4°C for 10 min.

### Example 2: Tail fiber Purification

The supernatant from Example 1 was then loaded onto a Ni-NTA column (IMAC HisTrap FF 5 ml, GE-Healthcare, US) that was washed with 2 column volumes of $H_2O$, elution buffer (50 mM Tris/HCl (pH 7.0), 500 mM NaCl, 5% glycerol (v/v), 250 mM Imidazole, 1 mM TCEP), and washing buffer (50 mM Tris/HCl [pH 7.0], 500 mM NaCl, 5% glycerol (v/v), 25 mM Imidazole, 1 mM TCEP) and then loaded with 95% of buffer A (5MM Tris/HCl, 500 mM NaCl, 5% glycerol, pH 8) and 5% of buffer B (50 mM Tris/HCl, 500 mM NaCl, 5% glycerol, 500 mM imidazole, pH 8). The supernatant was loaded onto the column with a maximum speed of 1 ml/min. The column was then washed with 5 column volumes of washing buffer to get rid of nonspecific binders. The bound tail fiber proteins were eluted with a linear gradient from 25 mM to 250 mM Imidazol in 10 column volumes and thereby fractions were collected that were then pooled. Fractions were analyzed on a 12% SDS-PAGE gel and pooled. Proteins were concentrated using 10 kDa MWCO Amicon Ultra centrifugal fitters according to the manufacturer's protocol (Merck, Germany).

### Example 3: Nanoparticle Preparation

In preparation for modification of the nanoparticles with the isolated bacteriophage tail fiber protein T7p52, 50 ml of 10 nM AuNPs were dissolved in 20 mg BSPP (Bis(p-sulfonatophenyl)phenylphosphine dihydrate dipotassium salt) with stirring for 3 days at room temperature. A 5 M NaCl solution was then added until the color of the solution turned blue. The suspension was then centrifuged at 1,600 × g at room temperature for 30 minutes and the supernatant was discarded. The pellet was resuspended in 800 μl of BSPP solution (2.5 mM in $H_2O$). An equal volume of methanol was then added. This suspension was centrifuged at 1,600 × g at room temperature for 30 min. The supernatant was discarded and the pellet was resuspended in 800 μl of BSPP solution (2.5 mM in $H_2O$). The concentration of AuNPs was determined by absorption spectroscopy at a wavelength of 518 nm and using an extinction coefficient of $8.5 × 10^7$ cm$^{-1}$ mol$^{-1}$.

**Example 4: Nanoparticle Coating**

To coat the AuNPs with the prepared tail fiber proteins that comprise an N-terminal cysteine and a His tag, the protein solution obtained in Example 2 was mixed with TCEP (final concentration 10 mM) and incubated for at least 30 minutes. Then, the AuNPs were mixed with a 100-fold excess of the modified viral tail fiber protein, followed by addition of sodium citrate buffer to a final concentration of 20 mM and pH of 3. After 3 minutes, HEPES buffer was added at a final concentration of 20 mM with pH 7. To remove unbound proteins, a 100 kDa MWCO centrifugal filter (Amicon Ultra, Merck Millipore) was used. The filters were filled with 400 μl 0.5x TBE and centrifuged at 2,000 rcf for 5 min. The filter was then loaded with the nanoparticle suspension and centrifuged at 10,000 rcf for 10 min.

**Example 5: Bacteria Preparation**

For the determination of the binding of the AuNPs coated with the T7p52 tail fiber proteins to the specific *E. coli* strain, a solution comprising the bacterium had to be obtained. For this, an overnight culture of the host bacterium ATCC 11303™ was initiated, resulting in an approximate bacterium concentration of $6 × 10^9$ CFU/ml the next day.

**Example 6: Tail fiber Binding Determination**

Two tubes containing approximately 1 ml coated nanoparticles as prepared in Example 4 in 1.5 ml of 1x PBS (8 g/L NaCl, 2 g/L KCl, 1.42 g/ L $Na_2HPO_4$, 0.27 g/L $K_2HPO_4$, pH 6.8-7.0) were prepared. Approximately 100 μl ATCC 11303™ bacteria from the overnight culture of Example 5 were added to one of the tubes, and both tubes were mixed. The redshift in the tube in which coated nanoparticles were contacted with the bacteria was detectable after about 1 min by visual inspection (see Figure 1).
The application further contains the following items:

1. A method for determining binding of a molecule to a cell surface epitope, comprising the steps of:

   a) contacting a plasmonic nanoparticle coated with a molecule capable of binding a cell surface epitope with a cell in an aqueous solution to obtain a mixed solution;
   b) determining the absorption spectrum of the mixed solution;

   wherein a shift in the absorption spectrum indicates binding of the molecule to the cell surface epitope thereby reducing the distance between the coated plasmonic nanoparticles to allow for plasmon coupling.

2. The method of item 1, wherein the plasmonic nanoparticle is smaller than 1000 nm, preferably smaller than 200 nm.

3. The method of items 1 or 2, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

4. The method of any one of the preceding items, wherein the molecule capable of binding a cell surface epitope is a protein.

5. The method of item 4, wherein the protein is a recombinant protein.

6. The method of item 5, wherein the recombinant protein is an antibody or a tail fiber protein, preferably a tail fiber protein.

7. The method of item 6, wherein the recombinant tail fiber protein is a recombinant viral tail fiber protein.

8. The method of any one of the preceding items, wherein the cell is a eukaryotic cell or a prokaryotic cell, preferably a prokaryotic cell.

9. The method of item 8, wherein the prokaryotic cell is a bacterium.

10. The method of item 9, wherein the bacterium is a multidrug resistant bacterium.

11. The method of item 8, wherein the eukaryotic cell is a mammalian cell.

12. The method of item 11, wherein the mammalian cell is a mammalian cancer cell.

13. The method of any one of the preceding items, wherein the plasmonic nanoparticle coated with a molecule capable of binding a cell surface epitope is the only type of plasmonic nanoparticle needed for determining binding of the molecule to the cell surface epitope.

14. The method of any one of the preceding items, wherein the aqueous solution is a buffer.

15. The method of any one of the preceding items, wherein the shift in the absorption spectrum is a redshift.

16. The method of any one of the preceding items, wherein the absorption spectrum is determined by visual inspection or spectrophotometry, preferably spectrophotometry.

17. The method of any one of the preceding items, wherein the method does not involve surface-enhanced Raman spectroscopy

18. The method of any one of the preceding items, wherein the method does not involve measuring surface plasmon resonance.

19. Use of a plasmonic nanoparticle coated with a molecule capable of binding a cell surface epitope for determining the binding of the molecule to the cell surface epitope by determining plasmon coupling between the plasmonic nanoparticles.

20. The use of item 19, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

21. The use of items 19 or 20, wherein the molecule capable of binding a cell surface epitope is a protein.

22. The use of item 21, wherein the protein is a recombinant protein.

23. The use of item 22, wherein the recombinant protein is an antibody or a tail fiber protein, preferably a tail fiber protein.

24. The use of item 23, wherein the recombinant tail fiber protein is a recombinant viral tail fiber protein.

25. The use of any one of items 19 to 24, wherein the cell is a eukaryotic cell or a prokaryotic cell, preferably a prokaryotic cell.

26. The use of item 25, wherein the prokaryotic cell is a bacterium.

27. The use of item 26, wherein the bacterium is a multidrug resistant bacterium.

28. The use of item 27, wherein the multidrug resistant bacterium is a bacterium from the group consisting of Vancomycin-Resistant Enterococci, Methicillin-resistant Staphylococcus aureus, Extended-spectrum beta-lactamase producing Gram-negative bacteria, Klebsiella pneumoniae carbapenemase producing Gram-negative bacteria, Multidrug-resistant Gram-negative rods and Multidrug-resistant tuberculosis bacteria.

29. The use of item 25, wherein the eukaryotic cell is a mammalian cell.

30. The use of item 29, wherein the mammalian cell is a mammalian cancer cell.

31. The use of any one of items 19 to 30, wherein the plasmonic nanoparticle coated with a molecule capable of binding a cell surface epitope is the only type of plasmonic nanoparticle needed for determining binding of the molecule to the cell surface epitope.

32. The use of any one of items 19 to 31, wherein the use does not involve surface-enhanced Raman spectroscopy

33. The use of any one of items 19 to 32, wherein the use does not involve measuring surface plasmon resonance.

34. A method for determining specific bacteriophage candidates for antibiotic treatment, comprising the steps of:

a) contacting a plasmonic nanoparticle coated with a viral tail fiber protein with an aqueous solution containing target bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for antibiotic treatment of the target bacteria.

35. The method of item 34, wherein the method is for determining patient-specific bacteriophage candidates for personalized antibiotic treatment and comprises the steps of:

a) contacting a plasmonic nanoparticle coated with a viral tail fiber protein with an aqueous solution containing patient-derived bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for personalized antibiotic treatment of the patient.

36. The method of items 34 or 35, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

37. The method of any one of items 34 to 36, wherein the bacteria is a multidrug resistant bacterium.

38. The method of item 37, wherein the multidrug resistant bacterium is a bacterium from the group consisting of Vancomycin-Resistant Enterococci, Methicillin-resistant Staphylococcus aureus, Extended-spectrum beta-lactamase producing Gram-negative bacteria, Klebsiella pneumoniae carbapenemase producing Gram-negative bacteria, Multidrug-resistant Gram-negative rods and Multidrug-resistant tuberculosis bacteria.

39. The method of any one of items 35 to 38 wherein the patient is a human, a domestic animal, or a livestock animal.

40. The method of any one of items 34 to 39, wherein the shift in the absorption spectrum is a redshift.

41. The method of any one of items 34 to 40, wherein the absorption spectrum is determined by visual inspection or spectrophotometry, preferably spectrophotometry.

42. The method of any one of items 34 to 41, wherein the method does not involve surface-enhanced Raman spectroscopy.

43. The method of any one of items 34 to 42, wherein the method does not involve measuring surface plasmon resonance.

44. Kit for detection of plasmon coupling comprising:

- plasmonic particles coated with a molecule capable of binding a cell surface epitope,
- an aqueous solution.

45. The kit of item 44, wherein the kit further comprises a control, wherein the control is selected from the group consisting of

- uncoated plasmonic particles, and
- plasmonic particles coated with a molecule not capable of binding the cell surface epitope.

46. The kit of items 44 or 45, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

47. The kit of any one of items 44 to 46, wherein the molecule capable of binding a cell surface epitope is a protein.

48. The kit of item 47, wherein the protein is a recombinant protein.

49. The kit of item 48, wherein the recombinant protein is an antibody or a tail fiber protein, preferably a tail fiber protein.

50. The kit of item 49, wherein the recombinant viral protein is a viral tail fiber protein.

51. The kit of any one of items 44 to 50, wherein the cell is a eukaryotic cell or a prokaryotic cell, preferably a prokaryotic cell.

52. The kit of item 51, wherein the prokaryotic cell is a bacterium.

53. The kit of item 52, wherein the bacterium is a multidrug resistant bacterium.

54. The kit of item 51, wherein the eukaryotic cell is a mammalian cell.

55. The kit of item 54, wherein the mammalian cell is a mammalian cancer cell.

56. The kit of any one of items 44 to 55, wherein the aqueous solution is suitable for absorption spectrum measurements.

57. The kit of item 56, wherein the absorption spectrum measurements do not involve surface-enhanced Raman spectroscopy.

58. The kit of items 56 or 57, wherein the absorption spectrum measurements do not involve measuring surface plasmon resonance.

59. Plasmonic nanoparticle coated with a molecule capable of binding a cell surface epitope.

60. The plasmonic nanoparticle of item 59, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

61. The plasmonic nanoparticle of items 59 or 60, wherein the molecule capable of binding a cell surface epitope is a protein.

62. The plasmonic nanoparticle of item 61, wherein the protein is a recombinant protein.

63. The plasmonic nanoparticle of item 62, wherein the recombinant protein is an antibody or a tail fiber protein, preferably a tail fiber protein.

64. The plasmonic nanoparticle of item 63, wherein the recombinant tail fiber protein is a recombinant viral tail fiber protein.

65. The plasmonic nanoparticle of any one of items 59 to 64, wherein the cell is a eukaryotic cell or a prokaryotic cell, preferably a prokaryotic cell.

66. The plasmonic nanoparticle of item 65, wherein the prokaryotic cell is a bacterium.

67. The plasmonic nanoparticle of item 66, wherein the bacterium is a multidrug resistant bacterium.

68. The plasmonic nanoparticle of item 65, wherein the eukaryotic cell is a mammalian cell.

69. The plasmonic nanoparticle of item 68, wherein the mammalian cell is a mammalian cancer cell

70. The plasmonic nanoparticle of any one of items 59 to 69, wherein the plasmonic nanoparticle is non-magnetic.

**REFERENCES**

Barbu et al. (2016): Phage Therapy in the Era of Synthetic Biology. In: Cold Spring Harbor perspectives in biology 8 (10).

Goncalves (2014): Plasmonic nanoparticles: fabrication, simulation and experiments. In: Journal of Physics D: Applied Physics 47 (21).

Strydom and Witthuhn (2015): Listeria monocytogenes: A Target for Bacteriophage Biocontrol. In: Comprehensive Reviews in Food Science and Food Safety 14(6), p. 694-704.

Tan et al. (2011): Building plasmonic nanostructures with DNA. In: Nature Nanotechnology 6, p. 268-276.

**Claims**

1. A method for determining binding of a tail fiber protein to a cell surface epitope of a bacterium, comprising the steps of:

   a) contacting a plasmonic nanoparticle coated with a tail fiber protein with the bacterium in an aqueous solution to obtain a mixed solution;
   b) determining the absorption spectrum of the mixed solution;

   wherein a shift in the absorption spectrum indicates binding of the tail fiber protein to the cell surface epitope of the bacterium thereby reducing the distance between the coated plasmonic nanoparticles to allow for plasmon coupling.

2. The method of claim 1, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

3. The method of claims 1 or 2, wherein the bacterium is a multidrug resistant bacterium.

4. Use of a plasmonic nanoparticle coated with a tail fiber protein for determining the binding of the tail fiber protein to a cell surface epitope of a bacterium by determining plasmon coupling between the plasmonic nanoparticles.

5. The use of claim 4, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

6. The use of claims 4 or 5, wherein the bacterium is a multidrug resistant bacterium.

**7.** A method for determining specific bacteriophage candidates for antibiotic treatment, comprising the steps of:

a) contacting a plasmonic nanoparticle coated with a viral tail fiber protein with a solution containing target bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for antibiotic treatment of the target bacteria.

**8.** The method of claim 7, wherein the method is for determining patient-specific bacteriophage candidates for personalized antibiotic treatment and comprises the steps of

a) contacting a plasmonic nanoparticle coated with a viral tail fiber protein with a solution containing patient-derived bacteria to obtain a mixed solution; and
b) determining the absorption spectrum of the mixed solution,

wherein a shift in the absorption spectrum of the mixed solution indicates that the bacteriophage from which the viral tail fiber protein is derived is a bacteriophage candidate for personalized antibiotic treatment of the patient.

**9.** The method of claims 7 or 8, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

**10.** The method of any one of claims 7 to 9, wherein the patient is suffering from an infection with a multidrug resistant bacterium.

**11.** The method of claim 10, wherein the multidrug resistant bacterium is a bacterium from the group consisting of Vancomycin-Resistant Enterococci, Methicillin-resistant Staphylococcus aureus, Extended-spectrum beta-lactamase producing Gram-negative bacteria, Klebsiella pneumoniae carbapenemase producing Gram-negative bacteria, Multidrug-resistant Gram-negative rods and Multidrug-resistant tuberculosis bacteria.

**12.** Kit for detection of plasmon coupling comprising:

- plasmonic nanoparticles coated with a tail fiber protein,
- an aqueous solution.

**13.** The kit of claim 12, wherein the kit further comprises a control, wherein the control is selected from the group consisting of

- uncoated plasmonic nanoparticles, and
- plasmonic nanoparticles coated with a tail fiber protein with a specificity for a different cell surface epitope.

**14.** The kit of claims 12 or 13, wherein the plasmonic nanoparticle is selected from the group consisting of a gold nanoparticle, a silver nanoparticle, a copper nanoparticle, a zinc nanoparticle, an aluminum nanoparticle, a bismuth nanoparticle, an MXene nanoparticle, TiN nanoparticle, a tungsten nanoparticle and a platinum nanoparticle, optionally mixtures thereof.

**15.** The kit of any one of claims 12 to 14, wherein the aqueous solution is suitable for absorption spectrum measurements.

Fig. 1

Europäisches
Patentamt

European
Patent Office

Office européen
des brevets

# EUROPEAN SEARCH REPORT

Application Number

EP 23 15 6155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RINGACI A ET AL: "Phage-mimicking nanoagents for rapid depolymerase specificity screening against multidrug resistant bacteria", BIOSENSORS AND BIOELECTRONICS, ELSEVIER SCIENCE LTD, UK, AMSTERDAM , NL, vol. 213, 1 June 2022 (2022-06-01), XP087110623, ISSN: 0956-5663, DOI: 10.1016/J.BIOS.2022.114444 [retrieved on 2022-06-01] | 1-10, 12-15 | INV. G01N33/553 G01N33/569 |
| Y | * figures 1A,1B, * <br> * page 4, column 2, paragraph 2 * <br> * page 2, column 2, paragraph 6 – paragraph 7 * <br> * page 4, column 2, line 7, paragraph 1 – line 11 * <br> * page 2, column 1, paragraph 4 * <br> * page 7, left column, last para: section 5 * | 11 | |
| Y | Bharadwaj Alok ET AL: "Multidrug-Resistant Bacteria: Their Mechanism of Action and Prophylaxis", BioMed Research International, 5 September 2022 (2022-09-05), pages 1-17, XP093064526, DOI: 10.1155/2022/5419874 Retrieved from the Internet: URL:https://downloads.hindawi.com/journals /bmri/2022/5419874.pdf [retrieved on 2023-07-17] * the whole document * | 11 | TECHNICAL FIELDS SEARCHED (IPC) <br><br> G01N |

-/--

The present search report has been drawn up for all claims

| Place of search | Date of completion of the search | Examiner |
|---|---|---|
| The Hague | 17 July 2023 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ...............................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 15 6155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LI-LIN TAY ET AL: "Silica encapsulated SERS nanoprobe conjugated to the bacteriophage tailspike protein for targeted detection of Salmonella", CHEMICAL COMMUNICATIONS, vol. 48, no. 7, 1 January 2012 (2012-01-01), page 1024, XP055025346, ISSN: 1359-7345, DOI: 10.1039/C1CC16325F scheme 1; * abstract; figure 3 * | 1-15 | |
| A | US 2022/112469 A1 (CHEN IRENE [US] ET AL) 14 April 2022 (2022-04-14) * figure 1 * | 1-15 | |

TECHNICAL FIELDS SEARCHED (IPC)

The present search report has been drawn up for all claims

| Place of search | Date of completion of the search | Examiner |
|---|---|---|
| The Hague | 17 July 2023 | van der Kooij, M |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

## ANNEX TO THE EUROPEAN SEARCH REPORT
## ON EUROPEAN PATENT APPLICATION NO.

**EP 23 15 6155**

This annex lists the patent family members relating to the patent documents cited in the above-mentioned European search report.
The members are as contained in the European Patent Office EDP file on
The European Patent Office is in no way liable for these particulars which are merely given for the purpose of information.

**17-07-2023**

| Patent document cited in search report | Publication date | Patent family member(s) | Publication date |
|---|---|---|---|
| US 2022112469 A1 | 14-04-2022 | US 2022112469 A1 | 14-04-2022 |
| | | WO 2020131192 A2 | 25-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BARBU et al.** Phage Therapy in the Era of Synthetic Biology. *Cold Spring Harbor perspectives in biology,* 2016, vol. 8 (10
- **GONCALVES.** Plasmonic nanoparticles: fabrication, simulation and experiments. *Journal of Physics D: Applied Physics,* 2014, vol. 47 (21
- **STRYDOM ; WITTHUHN.** Listeria monocytogenes: A Target for Bacteriophage Biocontrol. *Comprehensive Reviews in Food Science and Food Safety,* 2015, vol. 14 (6), 694-704
- **TAN et al.** Building plasmonic nanostructures with DNA. *Nature Nanotechnology,* 2011, vol. 6, 268-276